Europäisches Patentamt

European Patent Office

Office européen des brevets

(19)

(11) Numéro de publication: **0 197 876**

**B1**

(12) **FASCICULE DE BREVET EUROPEEN**

(45) Date de publication du fascicule du brevet: **11.07.90**

(51) Int. Cl.⁵: **A 61 K 31/415, A 61 K 47/00**

(21) Numéro de dépôt: **86470004.2**

(22) Date de dépôt: **24.03.86**

(54) Composition thérapeutique utilisable en dermatologie contenant de l'imidazolidinylurée comme principe actif.

(30) Priorité: **26.03.85 FR 8504616**

(43) Date de publication de la demande:
**15.10.86 Bulletin 86/42**

(45) Mention de la délivrance du brevet:
**11.07.90 Bulletin 90/28**

(84) Etats contractants désignés:
**AT BE CH DE FR GB IT LI NL**

(56) Documents cités:
**EP-A-0 080 281**
**EP-A-0 143 109**
**FR-A-2 531 336**
**GB-A-2 108 840**

Le dossier contient des informations techniques présentées postérieurement au dépôt de la demande et ne figurant pas dans le présent fascicule.

(73) Titulaire: **ANBEN**
**2, Rond-point de l'Esplanade**
**F-67000 Strasbourg (FR)**

(72) Inventeur: **Andermann Bensimon, Claudine**
**5, rue des Jonquilles**
**F-68000 Colmar (FR)**

(56) Documents cités:

**IL FARMACO, édition practicum, no. 11, novembre 1983, pages 415-428; M.F. BOBIN et al.: "Etude de la conservation de la dihydroxyacétone en fonction de la formulation"**
**CHEMICAL ABSTRACTS, vol. 94, no. 13, 30 mars 1981, page 170, abrégé 96957g, Columbus, Ohio, US; H. BECRENS et al.: "The synergistic action of isothiazolone E imidazolidinylurea", &**
**Parfums, Cosmet, Aromes 1980, 30, 81-4, 87**

Courier Press, Leamington Spa, England.

EP 0 197 876 B1

**Description**

La présente invention a pur objet une composition thérapeutique utilisable en dermatologie, plus particulièrement pour le traitement des manifestations cutanées de l'acné.

On sait que la pathologie de l'acné se caractérise par une hypersécrétion des glandes sébacées, conduisant à terme à la formation de bouchons, appelés comédons, dans le canal pilosébacé. Ces comédons finissent par boucher ce canal, ce qui entraine par la suite la formation plus ou moins rapide de papules, de pustules et de kystes, qui s'infectent. Toutes ces manifestations constituent le syndrome acnéique.

Des compositions thérapeutiques pour traiter cette maladie sont connues dans la mesure où plusieurs agents actifs nouveaux ont été découverts dans les dernières années. Ils sont utilisés, soit par voie systémique, soit par voie locale. On peut mentionner les tétracyclines, certaines hormones progestatives, et les rétinoides parmi les traitements par voie orale, les traitements par voie locale ayant la préférence des spécialistes, en particulier ceux utilisant l'acide rétinoique, le péoxyde be benzoyle, l'érythromycine, la clindamycine, dans diverses formes pharmaceutiques, comme des crèmes, des lotions alcooliques ou non, des tampons ou des bâtonnets type rouge à lèvres. Le mécanisme d'action de la plupart de ces agents est fondé sur le principe d'une irritation cutanée pour éliminer la couche cornée du derme et éliminer les éléments obturant les canaux pilosébacés. Les anciens traitements à base de soufre étaient déjà fondés sur le principe d'une activité kératolytique et abrasive de la peau.

D'autres compositions pharmaceutiques, comme celles à base de tétracyclines, d'érythromycine, et de clyndamycine, utilisent les propriétés chimiothérapeutiques des antibiotiques pour supprimer la composante anti-infectieuse de l'acné.

Il a également été proposé par les documents GB—A—2108840 et EP—A—0080281 d'utiliser de l'imidazolidinylurée en thérapeutique, et plus particulièrement pour les traitements dermatologiques, en particulier de l'acné.

La pratique et les tests montrent que les résultats annoncés pour les compositions décrites dans ces documents ne sont pas obtenus. Ceci est lié à la spécificité du traîtement de l'acné.

En effet, dans le traitement de l'acné, un certain nombre de produits anti-bactériens (antibiotiques et antiseptiques) ont été utilisés dans le passé avec succès. Il s'agit plus particulièrement de dérivés des tetracyclines comme la Minocycline pour les antibiotiques, ou le peroxyde de benzoyle pour les antiseptiques non antibiotiques. Or, il ne suffit pas qu'un produit chimique soit antibactérien pour qu'il soit actif dans le traitement de l'acné. C'est ainsi qu'un grand nombre d'autres antibiotiques ont été testés sans succès dans le traitement de l'acné, alors qu'ils ont montré une activité antibactérienne in vitro et in vivo intéressante.

Cette différence s'explique par le fait qu'il faille une pénétration adéquate au niveau du site d'action, constitué par le follicule pilo-sébacé, et que peu de produits ou de compositions atteignent cette performance. Par ailleurs, il faut éviter qu'ils ne soient trop pénétrants, au risque d'être toxiques.

Tous ces traitements sont en fait entachés, soit de redoutables effets secondaires nécessitant une surveillance clinique très sévère, qu'il s'agisse d'irritations cutanées ou d'allergies cutanées comme pour l'acide rétinoique et le péroxyde de benzoyle, par exemple, soit de résistances croisées observées avec les antibiotiques par voie orale, par exemple l'érythromycine.

La présente invention a pour but de pallier ces inconvénients.

Elle a pour objet une composition thérapeutique utilisable pour le traitement des manifestations cutanées de l'acné, caractérisée en ce qu'elle comporte comme principe actif de l'imidazolidinylurée ou un dérivé d'imidazolidinylurée dispersé dans un véhicule hydro-acétonique contenant des agents de mise en suspension non lipidiques et un promoteur d'absorption, en l'occurrence le saccharinate d'alkyldiméthylbenzylammonium.

Conformément à une autre caractéristique de l'invention, la composition thérupeutique est essentiellement composée:

— d'imidazolidinylurée ou d'un dérivé d'imidazolidinylurée en tant que principe actif

— de saccharinate d'alkyldiméthylbenzylammonium en tant que promoteur d'absorption

— d'un dérivé polyoxyéthylénique de l'alcool laurique en tant qu'agent tensio-actif non ionique

— d'acétone et d'eau en tant que véhicule hydro-acétonique

— de polymère carboxyvinylique en tant qu'agent tixotropique.

Selon une autre caractéristique de l'invention la composition thérapeutique est constituée par une phase aqueuse ou acétonique contenant:

— 0,005 à 5% en poids d'imidazolidinylurée ou d'un dérivé d'imidazolidinylurée, en particulier la 1,1′-méthylène- bis (3-(3-(hydroxyméthyl)-2,4-dioxo-5-imidazolidinyl) urée, actif contre les principaux germes responsables de l'infection acnéique, à des concentrations très faibles. Le pH de ces produits doit être particulièrement adapté pour assurer, d'une part, une bonne activité antimicrobienne, et, d'autre part, une bonne tolérance cutanée ainsi qu'une pénétration favorable au niveau des glandes sébacées.

— 0,005 à 5% en poids de saccharinate d'alkyldiméthylbenzylammonium en tant que promoteur d'absorption capable d'acheminer favorablement le ou les agents thérapeutiques actifs responsables d'une des activités thérapeutiques, au site d'action, constitué par le follicule pileux, d'une part, et les glandes sébacées, d'autre part.

— 0,02% à 20% en poids d'un agent tensio-actif non ionique, choisi, de préférence, parmi les dérivés polyoxyéthyléniques de l'alcool laurique.

— 1 à 10% en poids d'acétone destiné à assécher la peau et à favoriser ainsi la pénétration de la composition thérapeutique.

— 0,1 à 10% en poids de polymère carboxyvinylique assurant une thixotropie adéquate au mélange.

On est donc en présence d'une composition thérapeutique présentant des caractéristiques physico-chimiques, pharmaceutiques et galéniques particulières et capable de traiter de manière efficace les signes cliniques de l'acné. Ainsi, la composition thérapeutique assure un nettoyage particulièrement efficace de tous les dépôts lipidiques incrustés dans les diverses formations cutanées et pilosébacées, assure une activité contre les papules et les pustules, les agents chimiques la composant agissant en tant qu'agents comédolytiques, et exerce une activité antibactérienne contre la composante infectieuse de l'acné.

Conformément à une variante de l'invention, la composition thérapeutique contient des agents anti-inflammatoires, tels que des substances non stéroidiennes, comme les dérivés de l'énoxolone ou du bufexamac, ou tels qu'une solution aqueuse composée par deux constituants actifs dans la proportion de 3/1, à savoir 5-chloro, 2-méthyl, 4-isothiazoline, 3-one complexé par du chlorure de magnésium et d'autre part, 2-méthyl, 4-isothiazoline, 3-one également complexé par du chlorure de magnésium.

Suivant une autre variante de l'invention, la composition thérapeutique contient du parfum ou une essence non allergénique.

Selon une autre caractéristique de l'invention, la composition thérapeutique se présente sous la forme d'un gel, d'une lotion alcoolique ou non, d'une crème, de tampons ou de bâtonnets.

Il est donc possible de faire des compositions actives sous forme de produits liquide, semi-solide ou solide.

Parmi les liquides, la forme la plus caractéristique est une lotion non alcoolique à base de polyols, par exemple. Parmi les formes semi-solides, on peut mentionner les crèmes dermiques (émulsion huile/eau ou eau/huile), des mousses aqueuses, etc.. Parmi les formes solides, une composition type bâtonnet rouge à lèvres a été réalisée.

Ces différentes formes de produits peuvent être illustrées par la description détaillée des exemples suivants, donnés à titre non limitatif.

Exemple 1
Gel dermique transparent comprenant:
— 0,6 g % de polymère carboxyvinylique
— 2 g % de silicate de magnésium-aluminium colloidal
— 1 g % de polymère d'oxyéthylène
— qsp PH 4,5 d'hydroxyde de sodium 1N
— 1 g % d'imidazolidinylurée
— 0,025 g % de saccharinate d'alkyldiméthyl-benzylammonium
— qsp 100 % d'eau purifiée

Exemple 2
Lotion alcoolique comprenant:
— 1 g % d'imidazolidinylurée
— 0,03 g % de saccharinate d'alkyldiméthyl-benzylammonium
— 4,9 g % de glycéride d'acide caprique/Peg-6 caprilique
— 1,5 g % de glycérine
— 35 g % d'eau purifiée
— 0,5 g % d'acide citrique
—qsp 100 % d'alcool 96
— parfum qsp

Exemple 3
Crème dermique comprenant:
— 1 g % d'imidazolidinylurée
— 0,025 g % de saccharinate d'alkyldiméthyl-benzylammonium
— 6 g % de lauryléther polyoxyéthylène (laureth-23)
— 2 g % de polymère carboxyvinylique
— 2 g % d'oxyde de titane
— 5 g % d'acétone
— qsp PH 4,5 de triéthanolamine
— qsp 100 % d'eau purifiée

Selon une autre caractéristique de l'invention, la composition thérapeutique sous forme de gel contient de 0,1 à 5% d'un agent gélifiant thixotrope pour obtenir une viscosité comprise entre 15000 et 80000 centipoises (15—80 Pa · s) à une température d'environ 25°C.

Enfin, d'après l'exemple 1 et selon une dernière caractéristique de l'invention, la composition thérapeutique sous forme de lotion contient des composés choisis parmi les lauryléthers polyoxyéthylènes, le carboxyméthylène ou la triéthanolamine dans des concentrations de préférence entre 0,5% et 2,5% pour obtenir une viscosité comprise entre 500 et 1000 centipoises (0,5—1 Pa · s).

En effet, si le produit est trop rigide, la composition sera difficile à étaler, et la pénétration des agents actifs sera médiocre. Inversement, si la composition à appliquer est trop fluide, le patient aura du mal à régler la quantité à distribuer à partir du récipient.

La composition thérapeutique selon l'invention a été administrée par application bi-quotidienne, sous forme de massage sur la peau atteinte de lésions acnéiques. Avec cette posologie, l'efficacité thérapeutique du traitement fut notable au bout de quelques semaines, sans que les malades aient eu à subir les inconvénients des traitements kératolyltiques classiques se manifestant après quelques jours de traitement par des éruptions ccutanées, des rougeurs, et divers autres désagréments préjudiciables à l'esthétique.

Des essais cliniques effectués en double insu et comparant la composition thérapeutique décrite dans la présente invention à une composition pharmaceutique renfermant un agent kératolytique, ont montré la supériorité de la composition de l'invention sur le traitement de comparaison en particulier du point de vue de la tolérance et du confort à l'application. L'essai thérapeutique le plus significatif a porté sur 43 malades atteints d'acné comédonienne ou inflammatoire: 23 malades ont été traités avec la composition de l'invention, 20 malades avec une composition classique contenant 10% en p/v de peroxyde de benzoyle, pendant une durée de 3 mois. L'efficacité des traitements a été évaluée de manière objective, en utilisant l'"acné score" comme critères d'évaluation: comptage du nombre de comédons, papules, pustules, nodules sur une même superficie pour tous les malades dans la zone cutanée la plus atteinte, en comparant leur nombre respectif avant et après traitement. Après 2 mois de traitement, les lésions comédoniennes, papuleuses et pustuleuses avaient diminué de 80% et 50%, respectivement.

La comparaison de la tolérance et du confort à l'application a été très en faveur de la composition de l'invention, dans la mesure où aucun des malades soumis au traitement de l'invention ne s'est plaint de brûlures dans les premiers jours d'application du traitement, alors que des irritations cutanées parfois intenses sont apparues dans le groupe de malades soumis au traitement kératolytique de comparaison.

La composition de l'invention peut également être associée à d'autres traitements antiacnéiques de façon à obtenir un effet additif, contrairement aux autres thérapeutique, devant être administrées seules.

Bien entendu, la présente invention n'est pas limitée aux compositions thérapeutiques décrites. Des modifications restent possibles, sans sortir pour autant du domaine de protection de l'invention.

**Revendications**

1. Composition thérapeutique pour le traitement des manifestations cutanées de l'acné, caractérisée en ce qu'un principe actif, l'imidazolidinylurée ou un dérivé d'imidazolidinylurée est dispersé dans un véhicule hydroacétonique contenant des agents de mise en suspension non lipidiques et un promoteur d'absorption, en l'occurrence le saccharinate d'alkyldiméthylbenzylammonium.

2. Composition thérapeutique selon la revendication 1, caractérisée en ce qu'elle est composée:
— d'imidazolidinylurée ou d'un dérivé d'imidazolidinylurée en tant que principe actif
— de sacchrinate d'alkyldiméthylbenzylammonium en tant que promoteur d'absorption
— d'un dérivé polyoxyéthylénique de l'alcool laurique en tant qu'agent tensio-actif non ionique
— d'acétone et d'eau en tant que véhicule hydro-acétonique
— de polymère carboxyvinylique.

3. Composition thérapeutique selon la revendication 2, caractérisée en ce qu'elle contient:
— 0,005 à 5% en poids d'imidazolidinylurée ou d'un dérivé d'imidazolidinylurée
— 0,005 à 5% en poids de saccharinate d'alkylméthylbenzylammonium

— 0,02 à 20% en poids d'un dérivé polyoxyéthylénique de l'alcool laurique
— 1 à 10% en poids d'acétone
— 0,1 à 10% en poids de polymère carboxyvinylique.

4. Composition thérapeutique selon l'une quelconque des revendications 1 à 3, caractérisée en ce qu'elle contient des agents anti-inflammatoires, tels que des substances non stéroidiennes comme les dérivés de l'énoxolone ou du bufexamac ou tels qu'une solution aqueuse composée par deux constituants actifs dans la proportion de 3/1, à savoir d'une part 5-chloro, 2-méthyl, 4-isothiazoline, 3-one complexé par du chlorure de magnésium et d'autre part 2-méthyl, 4-isothiazoline, 3-one également complexé par du chlorure de mangésium.

5. Composition thérapeutique selon l'une quelconque des revendications 1 à 4, caractérisée en ce qu'elle contient parfum ou une essence non allergénique.

6. Composition thérapeutique selon l'une quelconque des revendications 1 à 5, caractérisée en ce qu'elles se présente sous la forme d'un gel, d'une lotion alcoolique ou non, d'une crème, de tampons ou de bâtonnets.

7. Composition thérapeutique selon la revendication 6, caractérisée en ce que, dans sa présentation sous forme de gel, elle contient:
— 0,6 g % de polymère carboxyvinylique
— 2 g % de silicate de magnésium-aluminium colloidal
— 1 g % de polymère d'oxyéthylène
— qsp PH 4,5 d'hydroxyde de sodium 1N
— 1 g % d'imidazolidinylurée
— 0,025 g % de saccharinate d'alkyldiméthylbenzylammonium
— qsp 100 % d'eau purifiée.

8. Composition thérapeutique selon la revendication 6, caractérisée en ce que, dans sa présentation sous forme de lotion, elle contient:
— 1 g % d'imidazolidinylurée
— 0,03 g % de saccharinate d'alkyldiméthylbenzylammonium
— 4,9 g % de glycéride d'acide caprique/Peg-6 caprilique
— 1,5 g % de glycérine
— 35 g % d'eau purifiée
— 0,5 g % d'acide citrique
— qsp. 100 % d'alcool 96
— parfum qsp.

9. Composition thérapeutique selon l'une quelconque des revendications 6 et 7, caractérisée en ce que pour l'obtention d'un gel de viscosité comprise entre 15000 et 80000 centipoises (15—80 Pa · s) à une température d'environ 25°C, elle contient de 0,1 à 5% d'un agent thixotrope.

10. Composition thérapeutique selon l'une quelconque des revendications 6 et 8, caractérisée en ce que pour l'obtention d'une lotion de viscosité comprise entre 500 et 1000 centipoises (0,5—1 Pa · s), elle contient des composés choisis parmi les lauryléthers polyoxyéthylènes, le carboxyméthylène ou la triéthanolamine dans des concentrations de préférence entre 0,5% et 2,5%.

## Patentansprüche

1. Eine therapeutische Mischung für die Behandlung von Hautmanifestationen der Akne, dadurch gekennzeichnet, daß ein Wirkstoff, Imidazolidinylharnstoff oder ein Derivat von Imidazolidinylharnstoff, in einer hydroacetonischen Trägersubstanz dispergiert ist, die nichtlipidische Suspendiermittel sowie einen Resorptionspromotor enthält, im vorliegenden Fall Alkyldimethylbenzylammoniumsaccharinat.

2. Eine therapeutische Mischung gemäß Anspruch 1, die dadurch gekennzeichnet ist, daß sie sich folgendermaßen zusammensetzt:
— Imidazolidinylharnstoff oder ein Derivat von Imidazolidinylharnstoff als Wirkstoff,
— Alkyldimethylbenzylammoniumsaccharinat als Resorptionspromotor,
— ein Polyoxyethylenderivat von Laurylalkohol asl nichtionisches Netzhaftmittel,
— Aceton oder Wasser als hydroacetonische Träger susbtanz,
— Carboxyvinylpolymerisat.

3. Eine therapeutische Mischung gemäß Anspruch 2, die dadurch gekennzeichnet ist, daß sie folgendes enthält:
— 0,005 bis 5 Gewichtsprozent Imidazolidinylharnstoff oder von einem Derivat des Imidazolidinylharnstoffs,
— 0,005 bis 5 Gewichtsprozent Alkylmethylbenzylammoniumsaccharinat,
— 0,02 bis 20 Gewichtsprozent eines Polyoxyethylenderivats von Laurylalkohol,
— 1 bis 10 Gewichtsprozent Aceton,
— 0,1 bis 10 Gewichtsprozent Carboxyvinylpolymerisat.

4. Eine therapeutische Mischung gemäß einem der Ansprüche 1 bis 3, dadurch gekennzeichnet, daß sie entzündungshemmende Mittel enthält, so zum Beispiel nichtsteroide Substanzen wie die Derivate von Enoxolon oder von Bufexamac oder eine wässrige Lösung, die aus zwei Wirkstoffen im Verhältnis 3:1 besteht, nämlich einerseits aus 5-Chlor-2-methyl-4-isothiazolin-3-on, das durch Magnesiumchlorid in einen Komplex überführt ist, und andererseits 2-Methyl-4-isothiazolin-3-on, das ebenfalls durch Magnesiumchlorid in einen Komplex überführt ist.

5. Eine therapeutische Mischung gemäß einem der Ansprüche 1 bis 4, dadurch gekennzeichnet, daß sie ein Parfum oder eine nichtallergene Essenz enthält.

6. Eine therapeutische Mischung gemäß einem der Ansprüche 1 bis 5, dadurch gekennzeichnet, daß sie in Form eines Gels, einer alkoholischen oder nichtalkoholischen Lotion, einer Creme, von Tupfern oder von Stiften angeboten wird.

7. Eine therapeutische Mischung gemäß Anspruch 6, dadurch gekennzeichnet, daß sie in ihrer Darreichungsform als Gel folgendes enthält:
— 0,6 g % Carboxyvinylpolymerisat,
— 2 g % kolloidales Magnesiumaluminiumsilikat,
— 1 g % Oxyethylenpolymerisat,
— Natriumhydroxid 1N qsp pH-Wert 4,5,

— 1 g % Imidazolidinylharnstoff,
— 0,025 g % Alkyldimethylbenzylammoniumsaccharinat,
— gereinigtes Wasser qsp 100 %.

8. Eine therapeutische Mischung gemäß Anspruch 6, dadurch gekennzeichnet, daß sie in ihrer Darreichungsform als Lotion folgendes enthält:
— 1 g % Imidazolidinylharnstoff,
— 0,03 g % Alkyldimethylbenzylammoniumsaccharinat,
— 4,9 g % Caprinsäureglycerid/Caprin-Peg-6,
— 1,5 g % Glycerin,
— 35 g % gereinigtes Wasser,
— 0,5 g % Zitronensäure,
— Alkohol 96 qsp 100 %,
— Parfum qs.

9. Eine therapeutische Mischung gemäß einem der Ansprüche 6 und 7, dadurch gekennzeichnet, daß sie, um ein Gel mit einer Viskosität herzustellen, die bei einer Temperatur von ungefähr 25°C zwischen 15000 und 80000 Zentipoise (15 bis 80 Pa · s) liegt, 0,1 bis 5% eines Thixotropiermittels enthält.

10. Eine therapeutische Mischung gemäß einem der Ansprüche 6 und 8, dadurch gekennzeichnet, daß sie, um eine Lotion mit einer Viskosität herzustellen, die zwischen 500 und 1000 Zentipoise (0,5 bis 1 Pa · s) liegt, Verbindungen enthält, die unter den Lauryletherpolyoxyethylenen, Carboxymethylen und Triaethanolamin ausgewählt werden, und zwar vorzugsweise in Konzentrationen zwischen 0,5% und 2,5%.

## Claims

1. A therapeutic composition for the treatment of dermatological signs of acne, characterised in that an active ingredient, imidazolidinyl urea or a derivative of imidazolidinyl urea, is dispersed in a hydro-acetonic carrier containing non lipidic suspension agents, as well as an absorption promotor, such as alkyldimethylbenzylammonium saccharinate.

2. A therapeutic composition according to claim 1, characterised in that the composition contains:
— imidazolidinyl urea or a derivative of imidazolidinyl urea as an active ingredient
— alkyldimethylbenzylammonium saccharinate as an absorption promotor
— a polyoxyethylene derivative of lauric alcohol as a non-ionic surfactant
— acetone and water as a hydro-actonic carrier
— a carboxyvinylic polymer.

3. A therapeutic composition according to claim 2, characterised in that it contains:
— 0,005 to 5% by weight of imidazolidinyl urea or a derivative of imidazolidinyl urea
— 0,005 to 5% by weight of alkyldimethylbenzylammonium saccharinate
— 0,02 to 20% by weight of a polyoxyethylene derivate of lauric alcohol
— 1 to 10% by weight of acetone
— 0,1 to 10% by weight of a carboxyvinylic polymer.

4. A therapeutic composition according to any of claims 1 to 3, characterised in that it contains non steroidical anti-inflammatory agents, such as enoxolone or bufexamac, wherein an aqueous solution is composed by two constituents in the proportion of 3/1, such as 5-chloro, 2-methyl, 4-isothiazoline, 3-one complexed by magnesium chloride on one hand and 2-methyl, 4-isothiazoline, 3-one also complexed with magnesium chloride on the other hand.

5. A therapeutic composition according to any of claims 1 to 4, characterised in that it contains a flavoring agent or a non allergenic perfume.

6. A therapeutic composition according to any of claims 1 to 5, characterised in that it is formulated as a gel, an alcoholic and a non alcoholic lotion, a cream, a paper tissue or a stick.

7. A therapeutic composition according to claim 6, characterised in that the gel formulation contains:

— 0,6 g % of a carboxyvinylic polymer
— 2 g % of aluminium-magnesium silicate
— 1 g % of an oxyethylene polymer
— a sufficient quantity of sodium hydroxyde to reach a pH of 4,5
— 1 g % of imidazolidinyl urea
— 0,025 g % of alkyldimethylbenzylammonium saccharinate

— a sufficient quantity of purified water to obtain 100 g %.

8. A therapeutic composition according to claim 6, characterised in that the lotion formulation contains:

— 1 g % of imidazolidinyl urea
— 0,03 g % of alkyldimethylbenzylammonium saccharinate
— 4,9 g % of capric acid/caprillic PEG-6 glyceride
— 1,5 g % of glycerol
— 35 g % of purified water
— 0,5 g % of citric acid
— a sufficient quantity of alcohol 96° to obtain 100 g %
— a sufficient quantity of perfume.

9. A therapeutic composition according to any of claims 6 and 7, characterised in that, in order to achieve a viscosity of the gel comprised between 15 and 80 Pa · s at a temperature of 25°C, it contains from 0,1 to 5% of a thixotropic agent.

10. A therapeutic composition according to any of claims 6 and 8, characterised in that, in order to achieve a viscosity of the lotion comprised between 0,5 and 1 Pa · s, it contains compounds such as laurylether polyoxyethylene, a carboxylvinylic polymer, or triethanolamine at a concentration preferably between 0,5% and 2,5%.